# EUROPEAN PATENT APPLICATION

(11) **EP 2 283 839 A1**
(43) Date of publication of application: **16.02.2011**
(21) Application number: 09164115.9
(22) Date of filing: 30.06.2009
(51) Int. Cl.: A61K 31/202, A61K 31/557, A61P 7/02

(54) **Polyunsaturated hydroxylated molecule having an eze geometry and its use in therapy**

(71) Applicant: Institut National des Sciences Appliquées de Lyon, 69100 Villeurbanne (FR)
(72) Inventor: Guichardant, Michel, 69370 Saint Didier au Mont d'Or (FR); Chen, Ping, 69100 Villeurbanne (FR); Lagarde, Michel, 69150 Décines Charpieu (FR); Véricel, Evelyne, 69100 Villeurbanne (FR)
(74) Representative: Tetaz, Franck Claude Edouard

(57) **Abstract**

The present invention relates to a polyunsaturated fatty molecule having at least 3 conjugated double bonds with a characteristic *EZE* geometry, 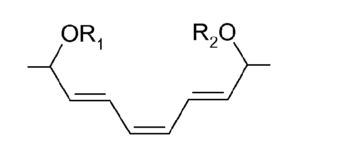 their use in therapy, particularly for the treatment of thrombosis and pharmaceutical compositions comprising the same.

## Description

### DOMAIN OF THE INVENTION

The present invention relates to a polyunsaturated fatty molecule having at least 3 adjacent double bonds with a characteristic *EZE* geometry, their use in therapy, particularly for the treatment of thrombosis and pharmaceutical compositions comprising the same.

### BACKGROUND OF THE INVENTION

Polyunsaturated fatty acids are molecules known in the art for their role for the balance of the human body, in several human body systems, such as the immune system or for the brain. Polyunsaturated fatty acids are also known for their use as diet supplements, some having been mentioned to reduce body weight and fat mass.

Polyunsaturated fatty acids having specific ZEE geometry have been shown to have potential role in inhibiting neuronal cells apoptosis (Lukiw & al.).

The inventors have now discovered that polyunsaturated fatty molecules and particularly fatty acids having a specific EZE geometry have unexpected anti-platelet aggregation.

The invention provides for new molecules having anti-platelet aggregation properties, particularly for their use in therapy in the treatment of thrombosis.

### BRIEF DESCRIPTION OF THE INVENTION

The present invention concerns a polyunsaturated fatty molecule comprising at least 3 conjugated double bonds of the following *EZE* structure: 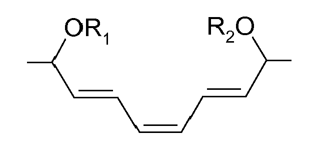 wherein
R1 and R2 independently represent a hydrogen or an C1-C3 alkyl,
and wherein the molecule is selected among the group consisting of fatty alcohols, fatty acids, fatty esters, fatty amines and fatty amides.

The polyunsaturated molecule can be represented preferably with the following formula I: 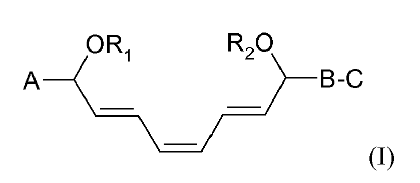 wherein
A and B combined with the *EZE* structure represent a polyunsaturated hydrocarbon chain of 16 to 24 carbon atoms,
C represent a terminal moiety selected among the group consisting of -OR3, -COR4 and - NR5R6,
R1 and R2 independently represent a hydrogen or a methyl,
R3 represents a hydrogen or a group -COR7,
R4 represents a group selected among -OR8, -NR9R10,
R5 and R6 independently represent a hydrogen or an C1-C6 alkyl or one of R5 or R6 represent a group -COR7,
R7 represents a C1-C6 alkyl,
R8 represents a hydrogen or a C1-C6 alkyl,
R9 and R10 independently represent a hydrogen or an C1-C6 alkyl,
and salts thereof.

In a preferred embodiment, the polyunsaturated molecule is represented with the following formula II 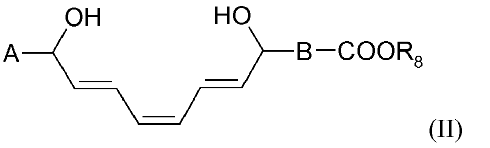 wherein
A and B combined with the *EZE* structure represent a polyunsaturated hycrocarbon chain of 16 to 24 carbon atoms and
R8 represents a hydrogen or a C1-C6 alkyl
and salts thereof.

The invention also concerns a method for the preparation of a polyunsaturated molecule having the characteristic *EZE* structure, comprising converting a polyunsaturated precursor of the n-3 or n-6 series selected among the group consisting of fatty alcohols, fatty acids, fatty esters, fatty amines and fatty amides by double oxygenation mechanism and recovering the polyunsaturated molecule having the required *EZE* structure: 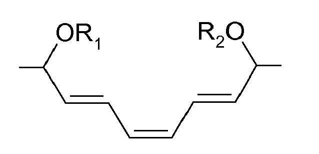 wherein
R1 and R2 independently represents a hydrogen or an C1-C3 alkyl.

The invention also concerns the polyunsaturated molecule of the invention for its use in therapy, particularly for its use in the treatment of platelet aggregation and more particularly thrombosis.

The invention also concerns a pharmaceutical composition comprising a polyunsaturated molecule of the invention having the characteristic *EZE* structure and a pharmaceutically acceptable vehicle.

### DETAILED DESCRIPTION OF THE INVENTION

In the present application, terms are employed with their usual meaning, except when précised otherwise.

"Polyunsaturated fatty molecule" means a hydrocarbon molecule having more than 10 carbon atoms, preferably more than 15 carbon atoms and 2 or more double bonds. In the present invention, the polyunsaturated fatty molecule has at least 3 conjugated double bonds having the required *EZE* structure. Fatty molecules have a hydrocarbon skeleton and can be linear, branched or cyclic, or at least parts of the hydrocarbon skeleton can be linear, branched or cyclic, and combinations thereof. In the present invention, the fatty molecules are selected among the group consisting of fatty alcohols, fatty acids, fatty esters, fatty amines and fatty amides which are well known in the art.

In the invention when the molecule is represented by one of formulas I or II:

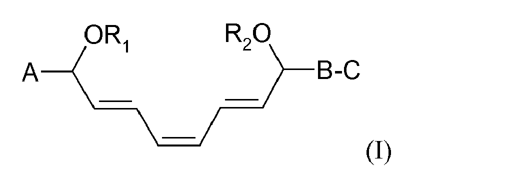

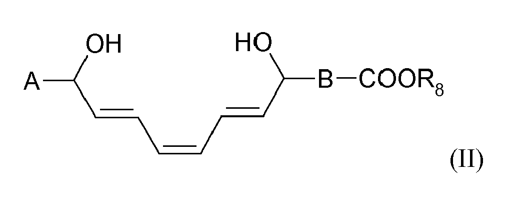

A and B independently represent preferably an alkyl, linear, branched or cyclic, saturated or unsaturated, having 2 to 14 carbon atoms.

The inventors found in a preferred embodiment that molecules wherein A and B are saturated alkyl moieties are more stable.

Particularly, the molecules of the invention are selected among the group consisting of:
- 10S,17S-dihydroxy-docosa-4*Z*,7*Z*,11*E*,13*Z*,15*E*,19*Z*-hexaenoic acid (PDX),
- 10(R),17(S)-dihydroxy-docosa-4*Z*,7*Z*,11*E*,13*Z*,15*E*,17*Z*-hexaenoic acid (10(R),17(S) diOH-22:6),
- 8(S),15(S)-dihydroxy-eicosa-5*Z*,9*E*,11*Z*,13*E*-tetraenoic acid (8(S),15(S) diHETE),
- 5(S),12(S)-dihydroxy-eicosa-6*E*,8*Z*,10*E*,14*Z*-tetraenoic acid (5(S),12(S) diHETE),
- 10(S),17(S)-dihydroxy-docosa-11*E*,13*Z*,15*E*,-trienoic acid (diOH-22:3),
   and salts thereof.

"Salts" are preferably pharmaceutically acceptable salts, such as pharmaceutically acceptable salt we preferably mean according to the invention a salt of a pharmaceutically acceptable acid or base, i.e. with any non-toxic acid or base, including the organic and inorganic acids or base. Such acids include acetic, benzenesulfonic, benzoic, citric, ethanesulfonic, fumaric, gluconic, glutamic, hydrobromic, hydrochloric, lactic, maleic, malic, mandelic, methanesulfonic, mucic, nitric, pamoic, pantothenic, phosphoric, succinic, sulfuric, tartaric and paratoluenesulfonic acid. Such bases are known in the art and include soda, primary, secondary or tertiary amines.

Methods for the preparation of the invention are known in the art and includes converting a polyunsaturated precursor of the n-3 or n-6 series selected among the group consisting of fatty alcohols, fatty acids, fatty esters, fatty amines and fatty amides by double oxygenation mechanism and recovering the polyunsaturated molecule having the required conjugated *EZE* structure: 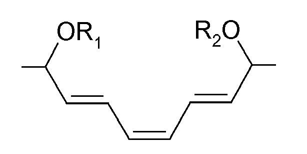 wherein
R1 and R2 independently represent a hydrogen or a methyl
When the molecule of the invention is a polyunsaturated fatty acid, it is obtained by double oxygenation of the corresponding fatty acid of the n-3 and n-6 series, particularly the acids 18:3n-6, 18:4n-3, 20:3n-6, 20:4n-6, 20:4n-3, 20:5n-3, 22:3n-6, 22:4n-6, 22:4n-3, 22:5n-6, 22:5n-3 and 22:6n-3.

More detailed disclosure of a method to obtain the claimed products is provided in the examples below.

The acid being obtained can be further transformed into the corresponding alcohol, amine, amide or ester as defined above by usual procedures known to the skilled person.

The molecules of the invention are used in therapy, particularly for its use in the treatment of platelet aggregation and more particularly thrombosis.

The skilled person will know how to administer the molecule of the invention to patient in need of a treatment at appropriate doses.

The following biological fatty acids are starting materials for obtaining the fatty molecules of the invention with the method of the invention:
- 16:3 n-6 hexadecatrienoic acid
- 18:3n-6 octadecatrienoic acid
- 18:4n-3 octadecatetraenoic acid
- 20:3n-6 eicosatrienoic acid
- 20:4n-6 eicosatetraenoic acid
- 20:4n-3 eicosatetraenoic acid
- 20:5n-3 eicosapentaenoic acid
- 22:3n-6 docosatrienoic acid
- 22:4n-6 docosatetraenoic acid
- 22:4n-3 docosatetraenoic acid
- 22:5n-6 docosapentaenoic acid
- 22:5n-3 docosapentaenoic acid
- 22:6n-3 docosahexaenoic acid

It was found that the molecules of the invention are active against platelet aggregation at submicromolar concentrations.

The invention also concerns a pharmaceutical composition comprising a polyunsaturated molecule of the invention having the characteristic conjugated *EZE* structure as active ingredient and a pharmaceutically acceptable vehicle.

Pharmaceutical compositions are well known in the art and depend on the administration route appropriate for the treatment. These compositions can be formulated for administration to mammals, including humans. The posology varies depending on the treatment and depending on the particular disorder.

These compositions are prepared so that they can be administered preferably *per* os (by the alimentary route) or by inj ection intra venous or parenterally.

In the pharmaceutical compositions of the present invention for oral, sublingual, subcutaneous, intramuscular, intravenous, transdermal, local or rectal administration, the active ingredient can be administered in unit forms of administration, mixed with conventional excipients, to animals or to humans. The appropriate unit forms of administration comprise the forms by the oral route such as tablets, capsules, powders, granules and oral solutions or suspensions, sublingual and buccal forms of administration, subcutaneous, intramuscular, intravenous, intranasal or intraocular forms of administration and rectal forms of administration.

The polyunsaturated molecules of the invention can be used in therapy on their own, or in combination with at least one other active agent. These other active agents are in particular selected from active substances suitable for the treatment of inflammatory diseases and platelet aggregation. These other active substances may be adjuvants for improving the activity of the polyunsaturated molecule of the invention, or other active substances known for their use in the treatment of said disorders. Such active agents are familiar to a person skilled in the art, and are commercially available or are described in reference handbooks such as Le Dictionnaire Vidal, including ticlopidin and aspirin.

The present invention therefore also relates to a product containing a polyunsaturated molecule according to the invention and another active agent as a compound product for therapeutic use, simultaneously, separately or spread over time, and in particular in the treatment of platelet aggregation, particularly thrombosis.

### FIGURES

Figure 1 represents the molecular structure of PDX obtained from 22:6n-3.
Figure 2 represents the curve data obtained for platelet aggregation at various concentrations of PDX.

### EXAMPLES

### Biosynthesis of 10S,17S-dihydroxy-docosa-4Z,7Z,11E,13Z,15E,19Z-hexaenoic acid, called PDX.

Reactions were catalyzed by soybean lipoxygenase (sLOX type 1-B) in ice cold 0.02 M sodium-borate buffer, pH 9.0. 10 µmoles DHA in 100 µL ethanol were mixed with 48 mL buffer and treated with 10 mg of sLOX prepared in 2 mL cold buffer. After 30 min, hydroperoxides were reduced by adding 10 mg of NaBH₄ in 2 mL cold buffer for 15 min at 0°C. The reaction mixture was acidified to pH 3 with glacial acetic acid and extracted on a C₁₈ solid-phase cartridge previously equilibrated with 5 mL of ethanol and 10 mL of deionized water. Cartridges were washed with 10 mL of water and di-hydroxylated fatty acids were eluted by 10 mL of ethanol and further dried under a stream of nitrogen. They were dissolved in 200 µL ethanol and stored at -20 °C.

PDX was purified by reverse phase high performance liquid chromatography (RP-HPLC) on a Waters XBridge C18 column (4.6 x 250 mm, 3.5 µm) at 40°C. A linear gradient was applied with a flow rate of 1 mL/min using two solvents: solvent A acidified to pH 3 with acetic acid was a mixture of acetonitrile/water (10:90, v/v), and solvent B was acetonitrile. Solvent A was first pumped for 5 min, then solvent B was increased to 30 % at 10 min, 40 % at 30 min, 60 % at 50 min and 100 % at 65 min. Solvent B was hold for 20 min before the system return to the initial conditions. PDX was detected using a diode array detector at 270 nm, and collected.

### Measurement of platelet aggregation

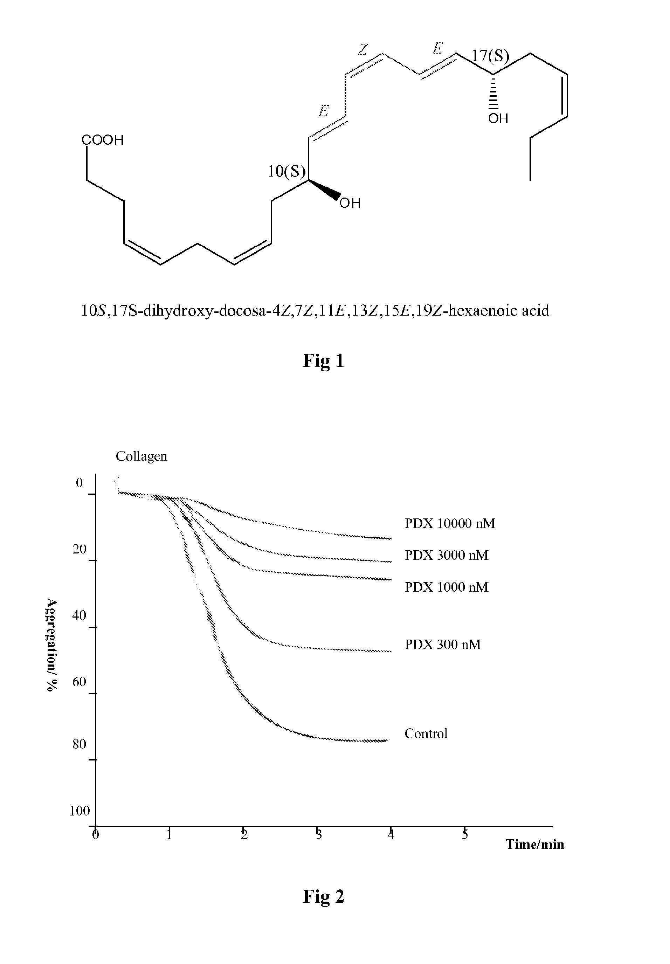

Aggregation tests were performed on platelets isolated from human blood in presence or not of different dihydroxylated triene compounds with different double bond geometry: ZEE, EZE or ZZZ. Platelet aggregation was determined by a standard turbidimetric method using a dual aggregometer (CHRONO-LOG, USA). The levels of light transmission were calibrated as 0 % for a platelet suspension and 100% for the Tyrode/HEPES solution (pH 7.35). 400 µl of platelet suspension in a vial was preincubated for 1 min at 37 °C under a continuous stirring condition, 1 µl of sample in ethanol was added for incubation, the activators (collagen, arachidonic acid, or U46619) were added after 2 min of incubation and the extent of platelet aggregation was measured after 4 min running.
The results obtained for different concentrations of PDX are given in Figure 2.
Other compounds were prepared using the same procedure or by conversion of PDX and compared to similar compounds of different configurations ZEE or EEE. Results are provided in the Tables I below.

**Table I: Effects of 1 µM triene molecules on Collagen-induced platelet aggregation**

| % inhibition | | ***E*/*Z*/*E*** | | | | |
|---|---|---|---|---|---|---|
| Control | | **PDX** | **10(R),17(S)-diOH-22:6** | **diOH-22:3** | **8(S),15(S)-diHETE** | **5(S),12(S)-diHETE** |
| 0.0 | | 56.1** | 70.0** | 63.8** | 66.5** | 74.9** |
| | | | | | | |

| | | | | | | |
|---|---|---|---|---|---|---|
| % inhibition | ***Z*/*E*/*E*** | | | | ***ElElE*** | |
| Control | **LTB₄** | **12-epi LTB₄** | **LTB₃** | **12-epi LTB₃** | **6-trans LTB₄** | **6trans-12-epi LTB₄** |
| 0.0 | 3.4 | 3.9 | 5.0 | 7.4 | 8.1 | 1.8 |

### E/Z/E

PDX: 10(S),17(S)-dihydroxy-docosa-4*Z*,7*Z*,11*E*,13*Z*,15*E*,17*Z*-hexaenoic acid
10(R),17(S)-diOH-22:6: 10(R),17(S)-dihydroxy-docosa-4*Z*,7*Z*,11*E*,13*Z*,15*E*,17*Z-*hexaenoic acid
diOH-22:3: 10(S),17(S)-dihydroxy-docosa-11*E*,13*Z*,15*E*,-trienoic acid
8(S),15(S)-diHETE: 8(S),15(S)-dihydroxy-eicosa-5*Z*,9*E*,11*Z*,13*E*
-tetraenoic acid 5(S),12(S)-diHETE: 5(S),12(S)-dihydroxy-eicosa-6*E*,8*Z*,10*E*,14*Z* tetraenoic acid

### Z/E/E

LTB₄: 5(S),12(R)-dihydroxy-eicosa-6*Z*,8*E*,10*E*,14*Z*-tetraenoic acid
12-epi LTB₄: 5(S),12(S)-dihydroxy-eicosa-6*Z*,8*E*,10*E*,14*Z*-tetraenoic acid
LTB₃: 5(S),12(R)-dihydroxy-eicosa-6*Z*,8*E*,10*E*,-trienoic acid
12-epi LTR₃: 5(S),12(S)-dihydroxy-eicosa-6*Z*,8*E*,10*E*,-trienoic acid

### E/E/E

6-trans-LTB₄: 5(S),12(R)-dihydroxy-eicosa-6*E*,8*E*,10*E*,14*Z*-tetraenoic acid
6-trans-12-epi-LTB₄: 5(S),12(S)-dihydroxy-eicosa-6*E*,8*E*,10*E*,14*Z*-tetraenoic acid

### REFERENCE

- Lukiw WJ, Cui JG, Marcheselli VL, Bodker M, Botkjaer A, Gotlinger K, Serhan CN, Bazan NG., A role for docosahexaenoic acid-derived neuroprotectin D1 in neural cell survival and Alzheimer disease, J Clin Invest. 2005 Oct; 115(10):2774-83.

## Claims

1. A polyunsaturated fatty molecule comprising at least 3 adjacent double bonds of the following conjugated *EZE* structure: 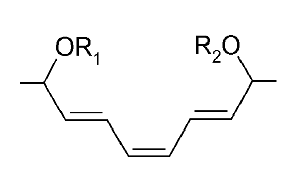 wherein
R1 and R2 independently represent a hydrogen or a methyl.
and wherein the molecule is selected among the group consisting of fatty alcools, fatty acids, fatty esters, fatty amines and fatty amides.

2. The polyunsaturated molecule of claim 1 having the following formula I: 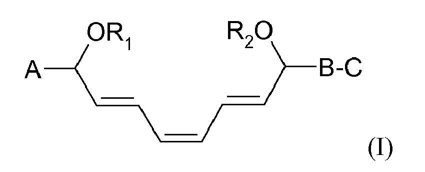 wherein
A and B combined with the *EZE* structure represent a polyunsaturated hydrocarbon chain of 16 to 24 carbon atoms,
C represent a terminal moiety selected among the group consisting of -OR3, -COR4 and - NR5R6,
R1 and R2 independently represent a hydrogen or an C1-C3 alkyl,
R3 represents a hydrogen or a group -COR7,
R4 represent a group selected among -OR8, -NR9R10,
R5 and R6 independently represent a hydrogen or an C1-C6 alkyl or one of R5 or R6 represent a group -COR7,
R7 represents a C1-C6 alkyl,
R8 represents a hydrogen or a C1-C6 alkyl,
R9 and R10 independently represent a hydrogen or an C1-C6 alkyl,
and salts thereof.

3. The polyunsaturated molecule of claim 2 having the following formula II: 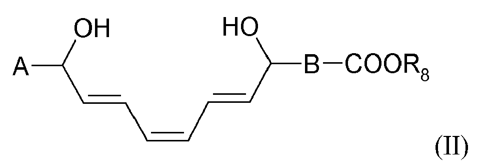 wherein
A and B combined with the conjugated *EZE* structure represent a polyunsaturated hydrocarbon chain of 16 to 24 carbon atoms and
R8 represents a hydrogen or a C1-C6 alkyl
and salts thereof.

4. The polyunsaturated molecule of one of claims 2 or 3, wherein
A and B independently represent an alkyl, linear, branched or cyclic, saturated or unsaturated, having 2 to 14 carbon atoms.

5. The polyunsaturated molecule of one of claims 2 to 4, wherein A and B are saturated alkyl moieties.

6. The polyunsaturated molecule of one of claims 1 to 5, selected among the group consisting of:
- 1S,17S-dihydroxy-docosa-4*Z*,7*Z*,11*E*,13*Z*,15*E*,19*Z*-hexaenoic acid (PDX),
- 10(R),17(S)-dihydroxy-docosa-4*Z*,7*Z*,11*E*,13*Z*,15*E*,17*Z*-hexaenoic acid (10(R),17(S) diOH-22:6)
- 8(S),15(S)-dihydroxy-eicosa-5*Z*,9*E*,11*Z*,13*E*-tetraenoic acid (8(S),15(S) diHETE)
- 5(S),12(S)-dihydroxy-eicosa-6*E*,8*Z*,10*E*,14*Z*-tetraenoic acid (5(S),12(S) diHETE)
- 10(S),17(S)-dihydroxy-docosa-11*E*,13*Z*,15*E*,-trienoic acid (diOH-22:3) and salts thereof.

7. A method for the preparation of a polyunsaturated molecule of one of claims 1 to 6, comprising converting a polyunsaturated precursor of the n-3 or n-6 series selected among the group consisting of fatty alcohols, fatty acids, fatty esters, fatty amines and fatty amides by double oxygenation mechanism and recovering the polyunsaturated molecule having the required structure: 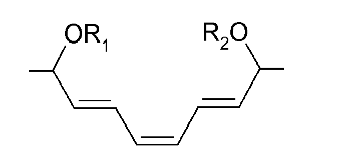 wherein
R1 and R2 independently represent a hydrogen or a methyl.

8. A polyunsaturated molecule of one of claims 1 to 6, for its use in therapy.

9. A polyunsaturated molecule of one of claims 1 to 6, for its use in the treatment of platelet aggregation and particularly thrombosis.

10. A pharmaceutical composition comprising a polyunsaturated molecule of one of claims 1 to 6 and a pharmaceutically acceptable vehicle.
